# EUROPEAN PATENT SPECIFICATION

(11) **EP 2 063 833 B1**
(45) Date of publication and mention of the grant of the patent: **26.03.2014**
(21) Application number: 07803580.5
(22) Date of filing: 21.09.2007
(51) Int. Cl.: A61F 13/00, A61F 13/02

(54) **A FILM DRESSING**
FOLIENVERBAND
ENDUIT DE PELLICULE

(30) Priority: 22.09.2006 DK 200601226
(43) Date of publication of application: 03.06.2009
(73) Proprietor: Coloplast A/S, 3050 Humlebæk (DK); Avery Dennison Corporation, Pasadena, CA 91103 (US)
(72) Inventor: FREDERIKSEN, Jesper Mads Bartroff, 2950 Vedbæk (DK); GEUENS, Bert, 2400 Mol (BE); DHAEZE, Kristine, 2230 Gierle (BE); EYCKENS, Luc, 2340 Beerse (BE)
(74) Representative: Inspicos A/S
(86) International application number: PCT/EP2007/060040
(87) International publication number: WO 2008/034897

(56) References cited:
- EP-A1- 1 181 930
- WO-A-97/43991
- WO-A2-02/47593
- DE-A1-102005 009 634
- US-A1- 2003 199 800

## Description

### INTRODUCTION

This invention relates to an adhesive flexible film suitable as a surgical drape or as a wound care dressing. In particular, the invention relates to a film dressing comprising a flexible film with opposite inner and outer surfaces, the inner surface being provided with an adhesive. A removable release liner is provided to protect the adhesive against undesired sticking to other items, and a removable support layer is bonded to the outer surface of the flexible film to support the flexible film and to maintain a smooth and un-wrinkled surface of the flexible film. The film dressing comprises a tab which is bonded to the support layer. The tab facilitates a users grip and thus facilitates a user in separating the support layer from the flexible film when the flexible film has been applied to a surface, e.g. to a wound of a patient. The invention further relates to a method of applying such a film dressing, to the use of such a film dressing in negative pressure wound treatment, and to a method of producing such a film dressing.

### BACKGROUND OF THE INVENTION

Flexible film dressings for wound treatment and in general for surgical drapes are known in the art. Typically, the known film dressings have a flexible film with a major surface coated or covered with pressure sensitive adhesive. A removable release liner covers the adhesive, and a flexible second film is bonded to the first film. The second film is suited to 'carry' the first film, making it easier to apply very thin, flexible and conformable first film coated with adhesive. The second, in general more rigid film is also called the support layer. After removal of the release liners, the construction is applied to the skin. In a second step, the support layer is removed while the first film remains adhered to the skin. Tabs, handle bars or similar application systems attached at the edge of the support layer to allow easy removal of the support layer are typically applied. A tab typically has an adhesive coated edge to attach the handle bar to the support layer, but can also have a non-adhesive coated part to act as a finger-lift. Other methods of sealing the tab to the support layer are possible. In prior art, tabs are mostly square or rectangular, laminated or otherwise constructed along the machine direction during conversion.

When the film dressing is applied, the first film is applied to the skin and the support layer is subsequently removed by pulling the tab. When pulling the tab, the first and second films must separate without lifting off the first film from the skin. It is important that the first flexible film stays completely adhered to the skin since wrinkles or air bubbles and channels between the skin and the first film may cause infection, maceration, or lead to decubitus. In particular in combination with negative pressure wound care, it is important to avoid separation of the first film from the skin of the patient, otherwise it will be difficult to sustain an adequate negative pressure beneath the film.

To prevent separation of the flexible film from the skin of a patient during separation of the support layer from the flexible film, one attempt has been to increase the bonding strength between the flexible film and the skin. This, however, may have a drawback in a later stage when the flexible film is to be removed from the skin, e.g. after ended treatment. Another known solution is to reduce the strength between the flexible film and the support layer. This, however, has the drawback that the support layer may separate from the flexible film too early and the flexible film may thereby wrinkle.

WO02/47593 discloses application devices comprising an applicator substrate, an interposed patch, and a release substrate. The document discloses that the application substrate and release substrate have graspable tabs allowing the user to grasp the tab of the applicator substrate, to grasp the tab on the release substrate, and to apply the patch to a target surface without releasing the tabs as originally grasped.

### DESCRIPTION OF THE INVENTION

It is an object of the invention to improve flexible film dressings and to facilitate separation of the support layer from the flexible film.

According to a first aspect, the invention provides a film dressing with a tab as specified in claim 1.

In a traditional system in which the support layer and tab are bonded along a straight edge, the force by which the flexible film and support layer are separated is essentially perpendicularly to the straight edge.

A non-linear interface zone may, during a users pull in the tab, cause pull directions which vary along the interface zone. Surprisingly, it has been found that such variations of the pull direction facilitate an easier separation of the support layer from the flexible film.

In this regards, a non-linear shape when used in this application, e.g. as describing the interface zone in the first aspect of the invention may embrace a wavy shape, a zigzag shape, or in general any shape which is not defined by one straight line, e.g. a sine-shape etc. furthermore, a non-linear shape may embrace more than one edge of the interface zone is non-linear. In one embodiment, the non-linear interface zone comprises one edge extending along a straight line and an opposite edge being non-linear.

The interface zone is defined as the zone in which the tab is bonded to the support layer. This bond may be continuous or discontinuous, i.e. broken into several bonded portions. The bond could be established by an adhesive, by welding the tab and support layer together, or the tab and support layer could be formed in one piece, e.g. from a single plate of a sheet material.

The interface could be located remote from edges of the film dressing. In this case, the non-linear shape could be provided by a non-linear adhesive layer or a non linear welding between the tab and the support layer. Alternatively, the interface could be located directly adjacent an edge of the film dressing, or at least adjacent an edge of the support layer and tab. This edge of the support layer, and tab or of the entire film dressing could be a non-linear edge, e.g. an edge having a shape which is similar to the shape of the interface zone.

In one particular embodiment, the interface zone forms a plurality of discontinuous bonded portions in which the support layer is attached to the tab and which are interrupted by non- bonded portions. Surprisingly, it has been found that such an interface zone allows for an even easier removal of the support layer after the flexible film is adhered to the skin, and the risk of lifting the flexible film off from the skin during removal of the support layer can therefore be further reduced. The bonded portions could be areas in which the tab is attached to the support layer e.g. by an adhesive, by welding etc, or the tab and support layer could be formed in one piece, e.g. from a piece of a sheet material in which a number of holes are punched out to form a plurality of areas which form transitions between the support layer portion of the sheet and the tab portion of the sheet. In this embodiment, the support layer portion of the sheet coextends the flexible film whereas the tab portion extends beyond the flexible film. The sheet could be folded in the transition between the tab and the support layer.

The flexible film could be made of polyurethane PU, PVC, EMA, silicone and other materials which are flexible and conformable to the skin, or the flexible film could be made of combinations between the mentioned materials, e.g. combinations between the mentioned materials and other materials.

Preferably, the flexible film is provided with a permeable structure allowing the skin to which the film is applied, to breath. On one surface, the film may be coated with an adhesive compound, e.g. a hydrocolloid adhesive, an acrylate adhesive, or a silicone adhesive. In one embodiment, the adhesive is breathable. On the opposite surface, the flexible film is in adhesive or electro static contact with a support layer which is preferably rigid relative to the flexible film. The support layer could be made from PE, PET, PP, paper, coated paper or other film materials that provide support to the flexible film. The support layer may be partly or fully coated with an adhesive to bond against the flexible film. The support layer may also provide dimensional stability during adhesion coating, conversion, assembly and handling. The flexible film could be laminated, cast, extruded or co-extruded onto the support film. In one embodiment, the support layer is less elastically deformable than the flexible film. The support layer could be provided with a thickness similar to the thickness of the flexible film or in the range of 0.5-10 times the thickness of the flexible film.

The removable release liner should protect the adhesive characteristics of the flexible film and could be of any kind known in the art.

The tab could be formed as a rectangular laminated or otherwise designed strip which is easily accessible for the user. To improve the users grip in the tab, the tab could be formed with a surface with a friction which is relatively large compared with the surface friction of the support layer. The tab may further be formed with protrusions, knobs, ribs or other structural features which enhance the users grip. The tab could also comprise a through hole enabling a solid finger grip, or the tab could be provided with a string which further facilitates gripping.

In order further to facilitate separation of the support layer from the flexible film, the interface zone could be arranged along a non-linear edge of at least one of the tabs and the support layer or along an edge formed by all layers of the film dressing, i.e. an edge formed by the release liner, the flexible film, the support layer and the tab. Again, non-linear may embrace e.g. a zigzagged edge or a wave-shaped edge. In this embodiment, it may be an advantage to provide the interface zone in the foremost projecting points of the non linear edge, i.e. in a part of the edge which is furthest away from a centre portion of the film dressing, e.g. in portions of the edge with a larger distance to a geometric centre of the film dressing while other portions with a shorter distance to the geometric centre of the film dressing could be un-bonded. If the edge is wave-shaped, the interface zone could be located at the wave crests while the non-bonded portions could be at the wave troughs.

The interface zone may e.g. constitute in the range of 10-90 percent of the total length of the non-linear edge. If the interface zone comprises discontinuous bonded portions, the above-mentioned range is the total length of all the portions relative to the total length of the non-linear edge.

The invention may facilitate the user to cut the original dressing into smaller dressings by providing the non-linear edge with a pattern of periodically repeated projections. As an example, the non-linear edge may form a wave-shaped pattern or a zigzagged pattern with repeated projections or wavy crests.

The interface zone may e.g. constitute in the range between 5 and 85 percent of the area of the waves or projections, e.g. in the range between 25 and 65 percent. This means that the tab is bonded to the support layer in an area which constitutes the above mentioned portions of the total area of the waves or projections. If the interface zone comprises discontinuous bonded portions, the sum of the bonded areas is within the above-mentioned areas of the total area of the waves or projections.

In one embodiment, the film dressing comprises at least two opposite non-linear or wave-shaped edges each forming an interface zone, e.g. with a plurality of discontinuous bonded portions. In another embodiment, the film dressing comprises at least two non-linear or wave-shaped interface zones located in the vicinity of opposite edges of the film dressing. The film dressing may further comprise one individual tab in connection with each wave-shaped interface zone.

An intermediate release liner may cover an intermediate portion of the adhesive and first and second edge liners may cover edge portions of the adhesive on opposite sides of the intermediate portion. The discontinuous bonded portions could be arranged along edges of the edge portions.

To facilitate that the separation of the support layer from the flexible film takes place from a narrow portion of the edge, e.g. from a corner point, the film dressing may have a tab with a protruding gripping element forming part of the free portion. As an example, the free portion may have a wave-shaped edge, or a protrusion arranged on the tab. In one embodiment, the film dressing comprises an attached string by which the user may grip the tab and use it for separation of the support layer from the flexible film.

In another embodiment of the invention, the tab comprises two free portions extending in opposite directions from the interface zone.

Under certain conditions, it may happen that the adhesive provided on the flexible film come in contact with the tab or it may happen that the adhesive provided on the flexible film come in contact with the adhesive which bonds the tab to the support layer. Unfortunately, this would make it difficult for the user to separate the support layer from the flexible film. To avoid this potential problem, the film dressing may, on an inner surface of the tab which faces the support layer, comprise a non-stick surface portion on which the adhesive of the flexible film is prevented from adhering. The non stick surface portion could extend beyond the other layers of the film dressing, or at least extend beyond the flexible film and the adhesive provided between this film and the release liner. The non-stick surface portion could be directly adjacent the interface zone and it may comprise a layer of a material with a silicone coating.

In a second aspect, the invention provides a method of applying a film dressing to a surface, said method comprising the steps of: providing a film dressing in accordance with the above description, removing the release liner from the flexible film, applying the flexible film to the surface, and removing the support layer from the flexible film by a pull in the tab. The film dressing could be applied to a wound e.g. in connection with a negative pressure wound treatment as disclosed in EP0620720.

### DETAILED DESCRIPTION OF THE INVENTION

A preferred embodiment of the invention will be described in further details with reference to the drawing in which:
Fig. 1 illustrates a cross-section of a film dressing in accordance with the present invention, and
Figs. 2, 3 illustrate a film dressing according to the invention, and
Figs. 4-20 illustrate various embodiments of the invention.

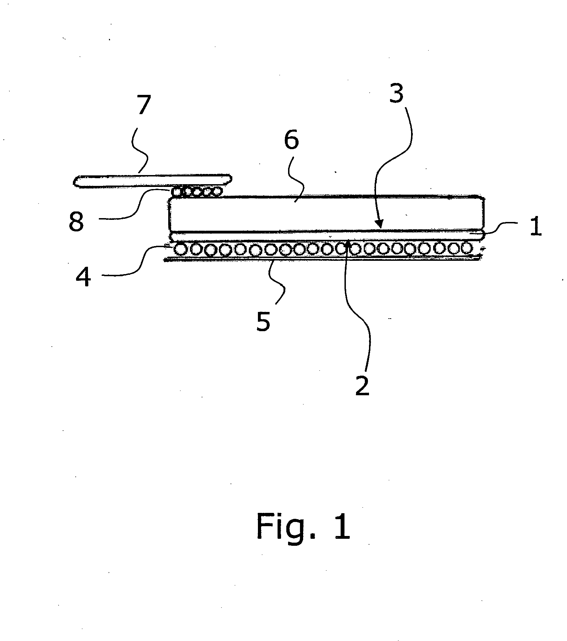

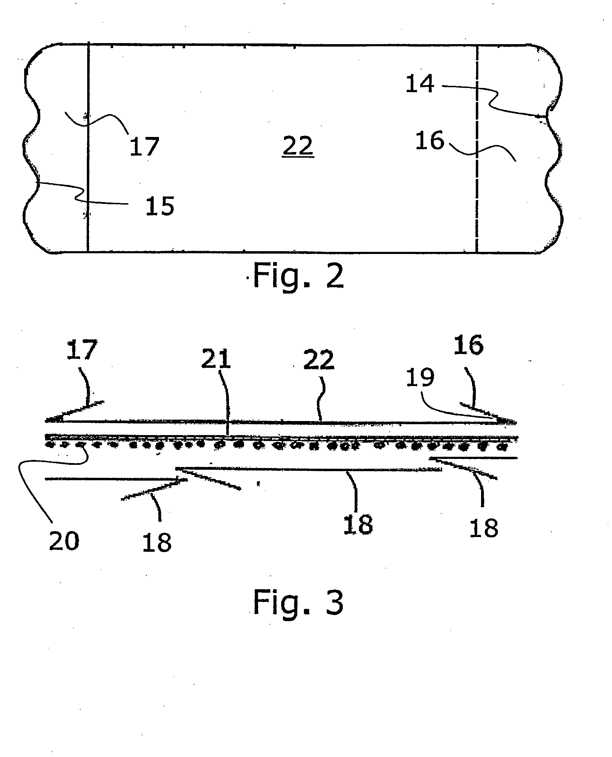

Referring to Fig. 1, the film dressing comprises a flexible film 1 with an inner surface 2 and an opposite outer surfaces 3. The inner surface is provided with an adhesive surface layer 4. A removable release liner 5 covers the adhesive and thus protects the adhesive surface until the film dressing is to be used. The removable support layer 6 is bonded to the first film, and stiffens the structure of the film dressing and thus facilitates the application of the flexible film to a wound. When the flexible film has been applied to the wound, the support layer is separated from the flexible film by a pull in the tab 7 which is attached to the support layer in an interface zone 8. As seen in Fig. 2, the interface zone is wave-shaped and thus non-linear.

The film dressing shown in a top view in Fig. 2 and in a cross-sectional view in Fig. 3 comprises two individual tabs 16, 17 which are attached to the support layer 22. The release liner 18 is split into an intermediate release liner covering an intermediate portion of the adhesive, and first and second edge liners covering edge portions of the adhesive on opposite sides of the intermediate portion. The interface zone has numeral 19, the adhesive provided on the flexible film has numeral 20, the flexible film has numeral 21, and the support layer has numeral 22. The tabs 16, 17 are bonded to the support layer in wave-shaped interface zone following the wave-shaped edges 14, 15 of the film dressing - i.e. in a non-linear shape.

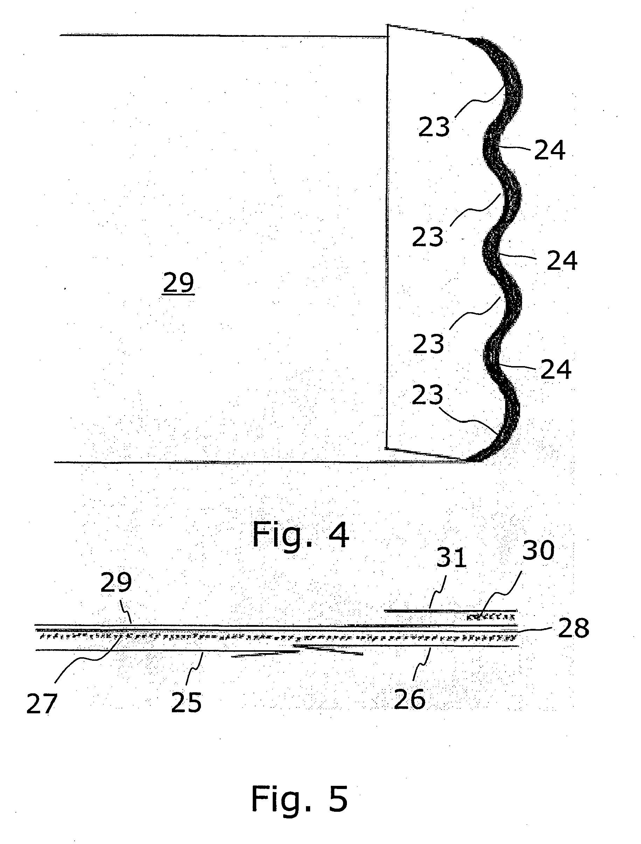

Figs. 4 and 5 illustrate an embodiment of the invention in which the interface zone has a non-linear, wave-shape with a repeated pattern of wave crests 23 and wave troughs 24.

Fig. 5 illustrates two release liners 25, 26, an adhesive 27, a flexible film polyurethane layer (PU layer) 28, a support polyethylene layer (PE layer) 29, an adhesive 30, and a tab 31. The tab 31 extends from an edge portion of the other layers towards a centre portion of the film dressing.

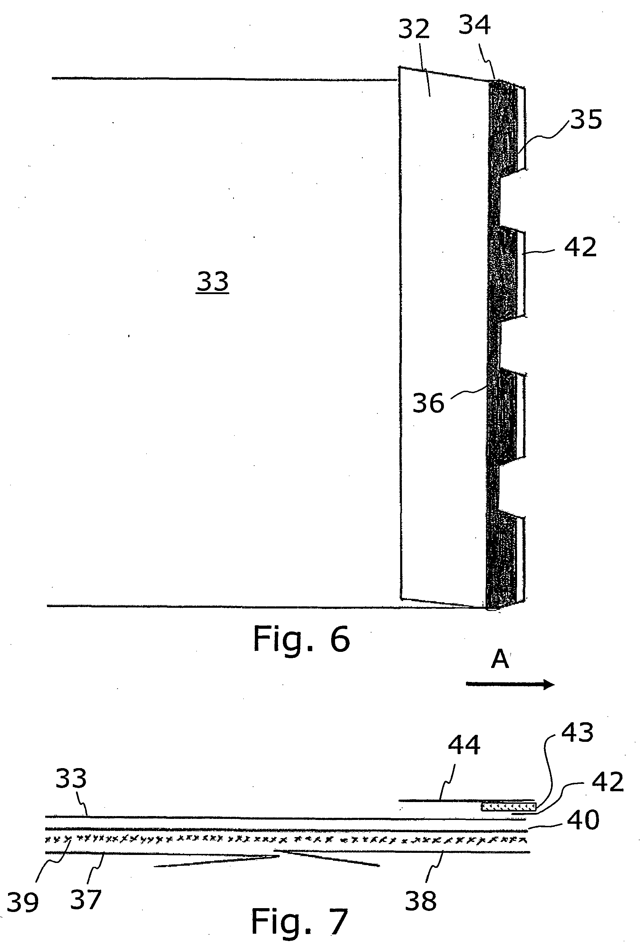

Figs. 6 and 7 illustrate another embodiment of a film dressing in which the tab 32 is bonded to the support layer 33 in an interface zone 34. The interface zone is considered to be non-linear since it comprises one side 35 having a non-linear shape, irrespective that the opposite side 36 has a linear shape.

The cross-sectional view in Fig. 7 illustrates two release liners 37, 38, an adhesive layer 39, a flexible film consisting of a PU layer 40, a support layer, i.e. a PE layer 33, a small layer of silicone 42, an adhesive layer 43, and a tab 44. The small label 42 comprises a layer of silicone providing a non-stick surface portion onto which the adhesive layer 39 can not adhere. The label 42, the adhesive layer 43 and the tab 44 extends beyond the other layers in the direction indicated by the arrow A. The non-stick surface portion thereby prevents contact between the adhesive 39 and the adhesive 43 and further prevents bonding of the tab onto the flexible film in case the adhesive layer 39 is deformed. Such a deformation may e.g. be caused by a cutting or stamping process in which the film dressing is punched out of a larger sheet, or floating of the adhesive during aging.

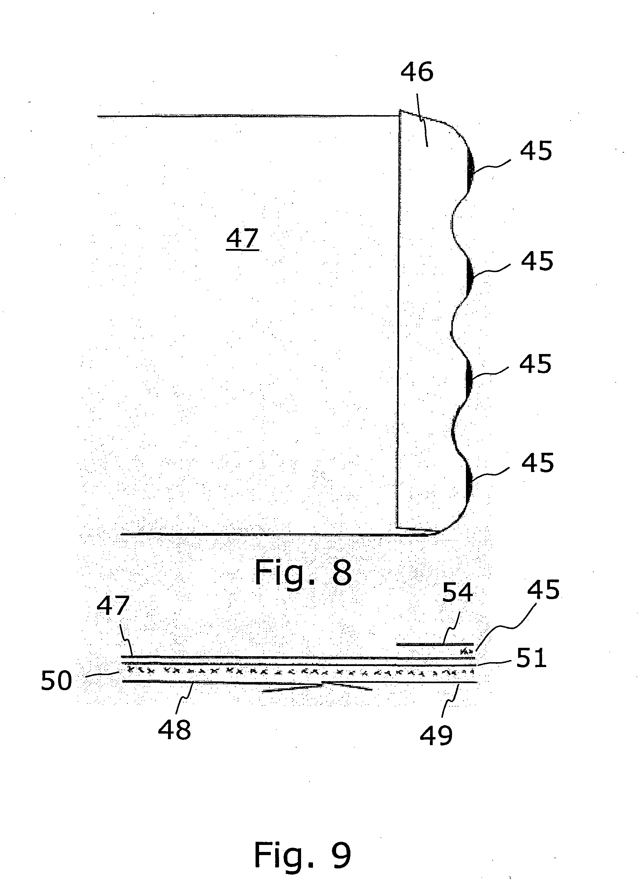

Figs. 8 and 9 illustrate an embodiment of the invention wherein the interface zone in which the tab 46 is bonded to the support layer 47 is wave-shaped like in Figs. 4, 5, but in which the interface zone comprises discontinuous bonded portions 45 located along the wave-shaped edge of the tab 46 and thus along the edge of the film dressing in its entirety.

The cross-sectional view in Fig. 9 illustrates two release liners 48, 49, an adhesive layer 50, a flexible film 51 made of PU, a support layer 47 made of PE, an adhesive layer forming the interface zone 45, and a tab 54.

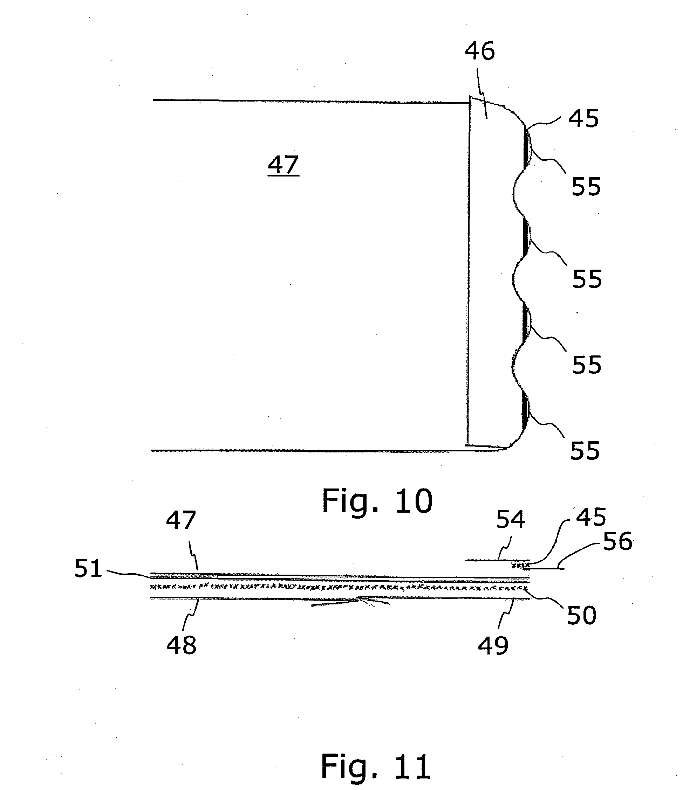

Figs. 10 and 11 illustrate an embodiment of the invention similar to the one illustrated in Figs. 8 and 9 but comprising non-stick portions 55 provided with layers of silicone 56, c.f. also the description of Fig 7. In this embodiment, only the non-stick portions extend beyond the other layers of the film dressing.

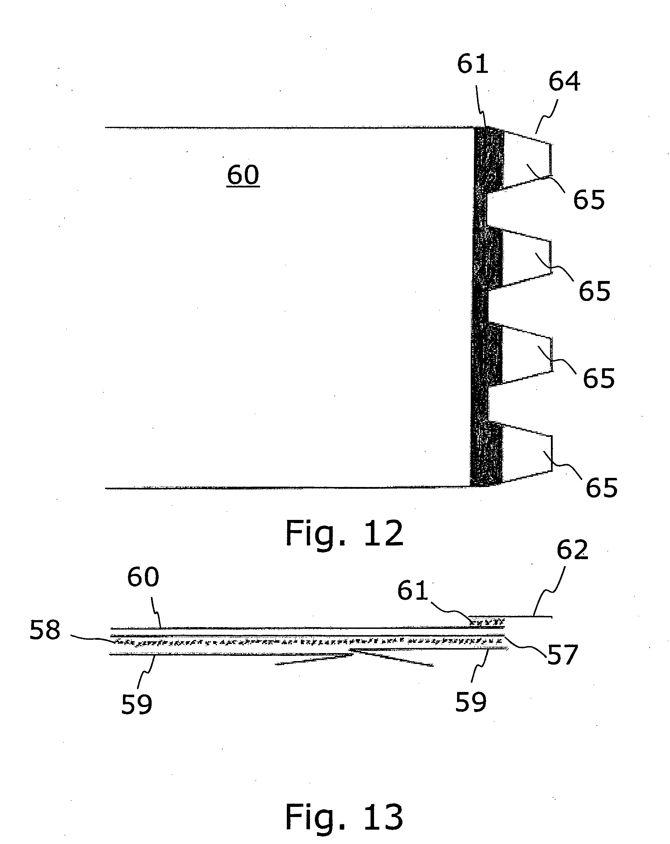

Figs. 12 and 13 illustrate an embodiment of the invention in which the film dressing comprises a flexible film 57 with opposite inner and outer surfaces, an adhesive 58 provided on the inner surface, a removable release liner 59 (in two pieces) covering the adhesive 58, a removable support layer 60 which is bonded to the outer surface of the flexible film, and a tab which is bonded to the support layer in an interface zone 61. The tab comprises a free portion 62 extending from the interface zone and outwardly away from the remaining parts of the film dressing. The free portion 62 comprises a gripping edge 64 forming a plurality of protrusions 65 by which a user may easily grab the tab and provide a pull which is unevenly distributed over the interface zone.

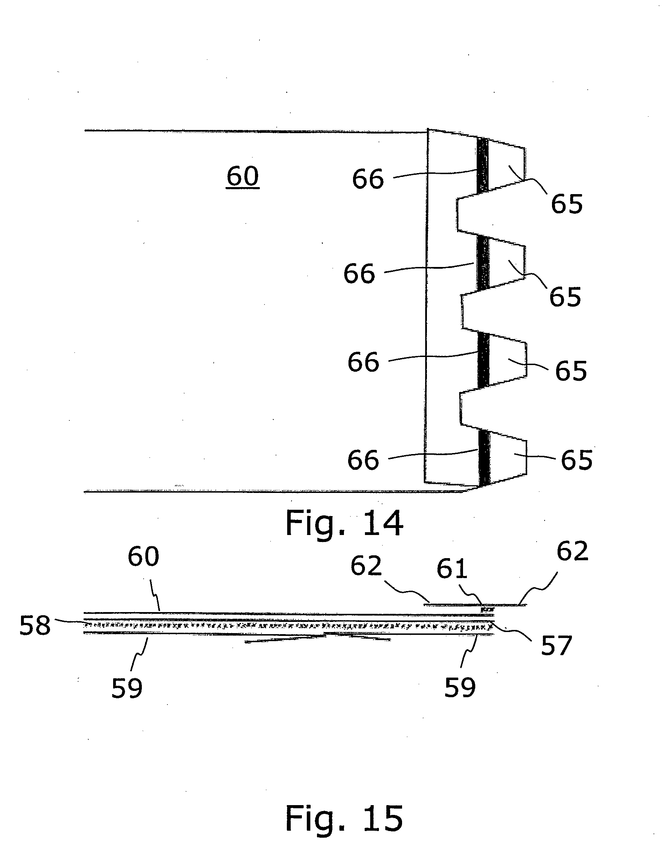

Figs. 14 and 15 illustrate a film dressing in which the interface zone comprises a plurality of discontinuous bonded portions 66. The bonded portions 66 (and thus the interface zone) are located intermediately between two free portions 62.

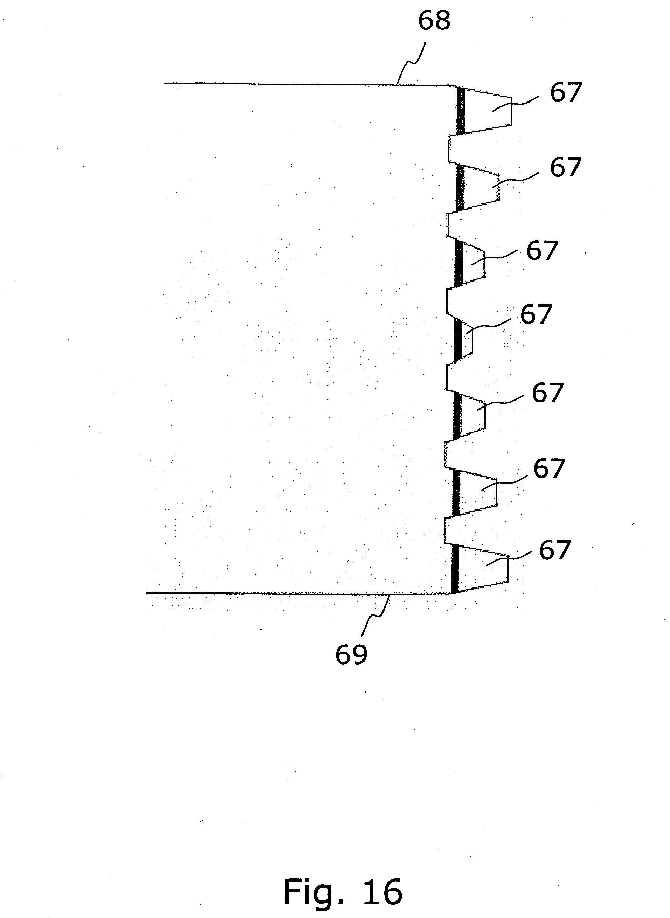

Fig 16 illustrates a film dressing similar to the one illustrated in Figs. 14 and 15 but wherein the plurality of protrusions 67 are of different size. The largest protrusions being located at the borders 68, 69 and the smallest intermediately there between. Naturally, the size could also decrease towards the borders so that the largest protrusion is in the middle between the borders.

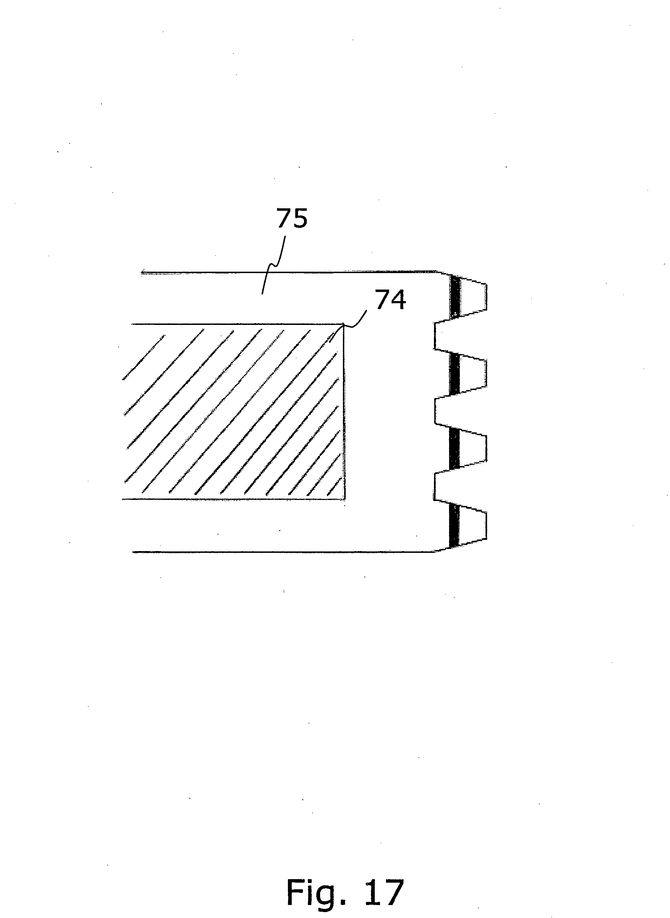

Fig. 17 illustrates a film dressing with a pad 74 of an absorbing material. The pad is arranged on the inner surface 75 of the flexible film to form an island wound dressing.

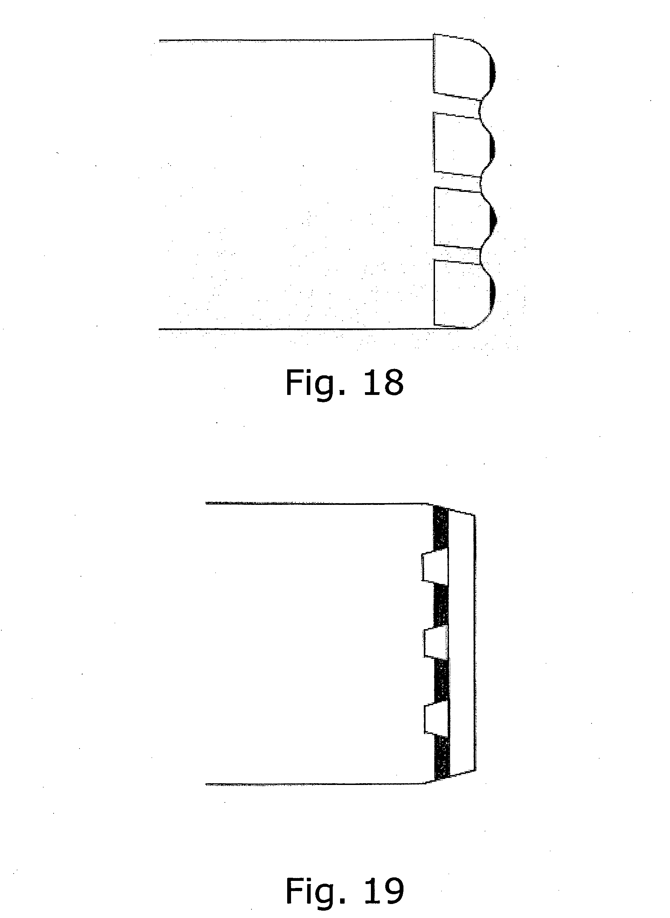

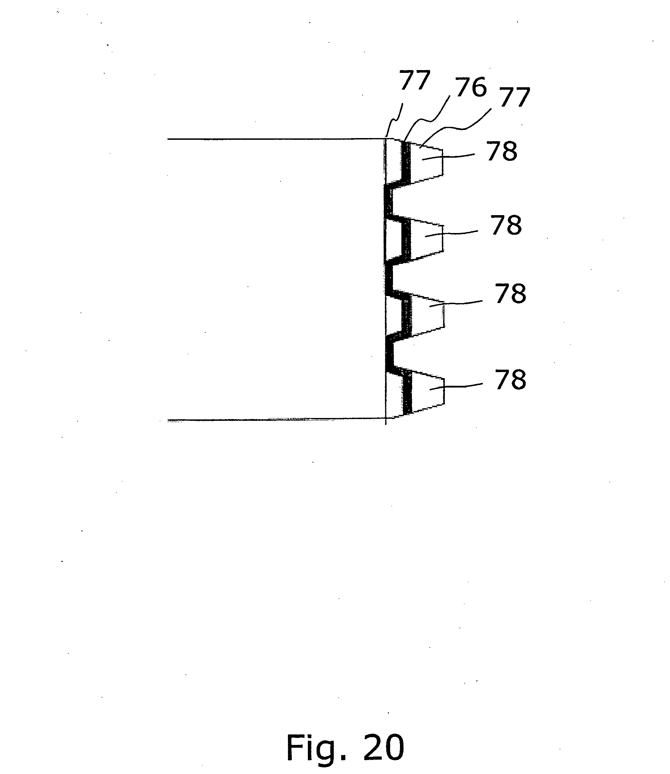

Figs. 18-19 illustrate further embodiments of the invention. The embodiment shown in Fig. 20 comprises an interface zone 76 which is non-linear, and a free portion 77 on both sides of the interface zone 76 and forming protrusions 78.

## Claims

1. A film dressing comprising
- a flexible film (1, 21, 28, 40, 51, 57) with opposite inner and outer surfaces (2, 3), the inner surface (2, 75) being provided with an adhesive (4, 20, 27, 39, 50, 58),
- a removable release liner (5, 18, 25, 26, 37, 38, 48, 49, 59) covering the adhesive (4, 20, 27, 39, 50, 58),
- a support layer (6, 22, 33, 47, 60) having an inner surface and an outer surface, the inner surface releasably bonded to the outer surface (3) of the flexible film (1),
- an interface zone (19, 34, 45, 66, 76) on the outer surface of the support layer, the interface zone (19, 34, 45, 66, 76) having a non-linear leading edge (14, 15, 35), and
- a tab (7, 16, 17, 31, 32, 44, 46, 54, 62),
**characterized in that** the tab (7, 16, 17, 31, 32, 44, 46, 54, 62) is bonded to the support layer (6, 22) in the interface zone (19, 34, 45, 66, 76) and comprises a free portion extending therefrom, the tab (7, 16, 17, 31, 32, 44, 46, 54, 62) being designed such that when a pull force is exerted in a non-linear direction along the leading edge (14, 15) of the interface zone, the support layer (6, 22) can release from the flexible film.

2. A film dressing according to claim 1, wherein the non-linear edge (14, 15, 35) is wave-shaped.

3. A film dressing according to claim 1, wherein a gripping edge (64) of the free portion of the tab (7, 16, 17, 31, 32, 44, 46, 54, 62) comprises at least one protrusion.

4. A film dressing according to claim 3, wherein the gripping edge (64) is wave-shaped.

5. A film dressing according to any of claims 1-4, wherein the bond between the support layer (6, 22, 33, 47, 60) and the tab (7, 16, 17, 31, 32, 44, 46, 54, 62) is discontinuous.

6. A film dressing according to any of the preceding claims, wherein the interface zone is adjacent an edge of at least one of the tab (7, 16, 17, 31, 32, 44, 46, 54, 62) and the support layer (6, 22, 33, 47, 60).

7. A film dressing according to claim 6, wherein the edge of at least one of the tab (7, 16, 17, 31, 32, 44, 46, 54, 62) and the support layer (6, 22, 33, 47, 60) is a non-linear edge.

8. A film dressing according to claim 6 or 7, wherein the interface zone (19, 34, 45, 66, 76) constitutes at between 30 and 70 percent of a length of the edge (14, 15, 35).

9. A film dressing according to any of the preceding claims, comprising at least two individual tabs (7, 16, 17, 31, 32, 44, 46, 54, 62) bonded to the support layer (6, 22, 33, 47, 60) along at least two interface zones located on the outer surface of the support layer in the vicinity of opposite edges of the film dressing.

10. A film dressing according to any of the preceding claims, comprising a free portion extending in two opposite directions from the interface zone (19, 34, 45, 66, 76).

11. A film dressing according to any of the preceding claims, comprising a pad of an absorbing material arranged on the adhesive covered inner surface of the flexible film (1, 21, 28, 40, 51, 57) to form an island wound dressing.

12. A film dressing according to any of the preceding claims, wherein a portion of an inner surface of the tab (7, 16, 17, 31, 32, 44, 46, 54, 62) which faces the support layer (6, 22, 33, 47, 60) comprises a non stick surface portion on which the adhesive (4, 20, 27, 39, 50, 58) of the flexible film do not adhere.

13. A film dressing according to claim 12 wherein the non-stick surface portion is between the interface zone and an edge of the film dressing.

14. A film dressing according to any of the claims 12-13, wherein the non-stick surface comprises a layer of silicone.

15. A film dressing according to any of the preceding claims, wherein the tab (7, 16, 17, 31, 32, 44, 46, 54, 62) comprises a string by which a user may pull the tab and thereby separate the support layer (6, 22, 33, 47, 60) from the flexible film.

16. A film dressing according to any of the preceding claims, further comprising a removable support layer having an inner surface and an outer surface, the inner surface which is bonded to the outer surface of the flexible film.

17. A method of applying a film dressing to a surface, **characterized in that** the method comprises the steps of:
- providing a film dressing in accordance with any of claims 1-16,
- removing the release liner (5, 18, 25, 26, 37, 38, 48, 49, 59) from the flexible film (1, 21, 28, 40, 51, 57),
- applying the flexible film (1, 21, 28, 40, 51, 57) to the surface, and removing the support layer (6, 22, 33, 47, 60) from the flexible film (1, 21, 28, 40, 51, 57) by a pull in the tab (7, 16, 17, 31, 32, 44, 46, 54, 62).

## Patentansprüche

1. Folienverband, der
- über eine flexible Folie (1, 21, 28, 40, 51, 57) mit einander gegenüberliegenden inneren und äußeren Oberflächen (2, 3), wobei die innere Oberfläche (2, 75) mit einem Klebemittel (4, 20, 27, 39, 50, 58) ausgestattet ist,
- über ein entfernbares Trennpapier (5, 18, 25, 26, 37, 38, 48, 49, 59), das das Klebemittel (4, 20, 27, 39, 50, 58) bedeckt,
- über eine Trägerschicht (6, 22, 33, 47, 60) mit einer inneren Oberfläche und mit einer äußeren Oberfläche, wobei die innere Oberfläche lösbar mit der äußeren Oberfläche (3) der flexiblen Folie (1) verbunden ist,
- über einen Zwischenbereich (19, 34, 45, 66, 76) auf der äußeren Oberfläche der Trägerschicht, wobei der Zwischenbereich (19, 34, 45, 66, 76) eine nichtgeradlinige Vorderkante (14, 15, 35) aufweist, und
- über eine Zunge (7, 16, 17, 31, 32, 44, 46, 54, 62) verfügt,
**dadurch gekennzeichnet, dass** die Zunge (7, 16, 17, 31, 32, 44, 46, 54, 62) mit der Trägerschicht (6, 22) in dem Zwischenbereich (19, 34, 45, 66, 76) verbunden ist und einen freien Abschnitt aufweist, der sich daraus erstreckt, wobei die Zunge (7, 16, 17, 31, 32, 44, 46, 54, 62) so ausgestaltet ist, dass, wenn eine Zugkraft in einer nichtgeraden Richtung entlang der Vorderkante (14, 15) des Zwischenbereichs ausgeübt ist, die Trägerschicht (6, 22) von der flexiblen Folie lösbar ist.

2. Folienverband nach Anspruch 1, bei dem die nichtgeradlinige Kante (14, 15, 35) wellenförmig ist.

3. Folienverband nach Anspruch 1, bei dem eine Greifkante (64) des freien Abschnitts der Zunge (7, 16, 17, 31, 32, 44, 46, 54, 62) wenigstens einen Vorsprung aufweist.

4. Folienverband nach Anspruch 3, bei dem die Greifkante (64) wellenförmig ist.

5. Folienverband nach einem der Ansprüche 1-4, bei dem die Verbindung zwischen der Trägerschicht (6, 22, 33, 47, 60) und der Zunge (7, 16, 17, 31, 32, 44, 46, 54, 62) diskontinuierlich ist.

6. Folienverband nach einem der vorangehenden Ansprüche, bei dem der Zwischenbereich benachbart einer Kante wenigstens einer der Zungen (7, 16, 17, 31, 32, 44, 46, 54, 62) und der Trägerschicht (6, 22, 33, 47, 60) liegt.

7. Folienverband nach Anspruch 6, bei dem die Kante wenigstens einer der Zungen (7, 16, 17, 31, 32, 44, 46, 54, 62) und der Trägerschicht (6, 22, 33, 47, 60) eine nichtgeradlinige Kante ist.

8. Folienverband nach Anspruch 6 oder 7, bei dem der Zwischenbereich (19, 34, 45, 66, 76) zwischen 30 und 70 Prozent einer Länge der Kante (14, 15, 35) bildet.

9. Folienverband nach einem der vorangehenden Ansprüche, der wenigstens zwei einzelne Zungen (7, 16, 17, 31, 32, 44, 46, 54, 62) aufweist, die mit der Trägerschicht (6, 22, 33, 47, 60) entlang wenigstens zweier Zwischenbereiche verbunden sind, die auf der äußeren Oberfläche der Trägerschicht in der Nähe der gegenüberliegenden Kanten des Folienverbands angeordnet sind.

10. Folienverband nach einem der vorangehenden Ansprüche, der einen freien Abschnitt aufweist, der sich in zwei entgegengesetzte Richtungen von dem Zwischenbereich (19, 34, 45, 66, 76) erstreckt.

11. Folienverband nach einem der vorangehenden Ansprüche, der ein Kissen aus einem absorbierenden Material aufweist, das auf der mit Klebemittel bedeckten inneren Oberfläche der flexiblen Folie (1, 21, 28, 40, 51, 57) angebracht ist, um einen Wundschnellverband zu bilden.

12. Folienverband nach einem der vorangehenden Ansprüche, bei dem ein Abschnitt einer inneren Oberfläche der Zunge (7, 16, 17, 31, 32, 44, 46, 54, 62), der der Trägerschicht (6, 22, 33, 47, 60) gegenüberliegt, einen nicht haftenden Oberflächenabschnitt aufweist, auf dem das Klebemittel (4, 20, 27, 39, 50, 58) der flexiblen Folie nicht klebt.

13. Folienverband nach Anspruch 12, bei dem der nicht haftende Oberflächenbereich zwischen dem Zwischenbereich und einer Kante des Folienverbands liegt.

14. Folienverband nach einem der Ansprüche 12-13, bei dem die nicht haftende Oberfläche eine Silikonschicht aufweist.

15. Folienverband nach einem der vorangehenden Ansprüche, bei dem die Zunge (7, 16, 17, 31, 32, 44, 46, 54, 62) einen Faden aufweist, mit dem ein Anwender die Zunge ziehen kann und damit die Trägerschicht (6, 22, 33, 47, 60) von der flexiblen Folie trennt.

16. Folienverband nach einem der vorangehenden Ansprüche, der weiterhin eine entfernbare Trägerschicht mit einer inneren Oberfläche und mit einer äußeren Oberfläche aufweist, wobei die innere Oberfläche mit der äußeren Oberfläche der flexiblen Folie verbunden ist.

17. Verfahren zum Aufbringen eines Folienverbands auf eine Oberfläche, **dadurch gekennzeichnet, dass** das Verfahren die Schritte aufweist:
- Bereitstellen eines Folienverbands nach einem der Ansprüche 1-16,
- Entfernen des Trennpapiers (5, 18, 25, 26, 37, 38, 48, 49, 59) von der flexiblen Folie (1, 21, 28, 40, 51, 57),
- Aufbringen der flexiblen Folie (1, 21, 28, 40, 51, 57) auf die Oberfläche und Entfernen der Trägerschicht (6, 22, 33, 47, 60) von der flexiblen Folie (1, 21, 28, 40, 51, 57) durch ein Ziehen an der Zunge (7, 16, 17, 31, 32, 44, 46, 54, 62).

## Revendications

1. Pansement comprenant un film, comportant :
- un film flexible (1, 21, 28, 40, 51, 57) comportant une surface interne et une surface externe opposées (2, 3), la surface interne (2, 75) étant pourvue d'un adhésif (4, 20, 27, 39, 50, 58),
- une doublure anti-adhésive amovible (5, 18, 25, 26, 37, 38, 48, 49, 59) recouvrant l'adhésif (4, 20, 27, 39, 50, 58),
- une couche de support (6, 22, 33, 47, 60) comportant une surface interne et une surface externe, la surface interne étant fixée de façon amovible à la surface externe (3) du film flexible (1),
- une zone d'interface (19, 34, 45, 66, 76) sur la surface externe de la couche de support, la zone d'interface (19, 34, 45, 66, 76) comportant un bord avant non linéaire (14, 15, 35), et
- une languette (7, 16, 17, 31, 32, 44, 46, 54, 62),
**caractérisé en ce que** la languette (7, 16, 17, 31, 32, 44, 46, 54, 62) est fixée à la couche de support (6, 22) dans la zone d'interface (19, 34, 45, 66, 76) et comprend une partie libre qui s'étend à partir de celle-ci, la languette (7, 16, 17, 31, 32, 44, 46, 54, 62) étant conçue de telle sorte que quand une force de traction est exercée dans une direction non linéaire le long du bord avant (14, 15) de la zone d'interface, la couche de support (6, 22) peut se détacher du film flexible.

2. Pansement comprenant un film selon la revendication 1, dans lequel le bord non linéaire (14, 15, 35) est en forme de vague.

3. Pansement comprenant un film selon la revendication 1, dans lequel un bord de préhension (64) de la partie libre de la languette (7, 16, 17, 31, 32, 44, 46, 54, 62) comprend au moins une partie saillante.

4. Pansement comprenant un film selon la revendication 3, dans lequel le bord de préhension (64) est en forme de vague.

5. Pansement comprenant un film selon l'une quelconque des revendications 1-4, dans lequel la liaison entre la couche de support (6, 22, 33, 47, 60) et la languette (7, 16, 17, 31, 32, 44, 46, 54, 62) est discontinue.

6. Pansement comprenant un film selon l'une quelconque des revendications précédentes, dans lequel la zone d'interface est adjacente à un bord d' au moins l'une de la languette (7, 16, 17, 31, 32, 44, 46, 54, 62) et de la couche de support (6, 22, 33, 47, 60).

7. Pansement comprenant un film selon la revendication 6, dans lequel le bord d'au moins l'une de la languette (7, 16, 17, 31, 32, 44, 46, 54, 62) et de la couche de support (6, 22, 33, 47, 60) est un bord non linéaire.

8. Pansement comprenant un film selon la revendication 6 ou 7, dans lequel la zone d'interface (19, 34, 45, 66, 76) constitue de 30 % à 70 % d'une longueur du bord (14, 15, 35).

9. Pansement comprenant un film selon l'une quelconque des revendications précédentes, comportant au moins deux languettes individuelles (7, 16, 17, 31, 32, 44, 46, 54, 62) fixées à la couche de support (6, 22, 33, 47, 60) le long d'au moins deux zones d'interface situées sur la surface externe de la couche de support à proximité de bords opposés du pansement comprenant un film.

10. Pansement comprenant un film selon l'une quelconque des revendications précédentes, comportant une partie libre qui s'étend dans deux sens opposés à partir de la zone d'interface (19, 34, 45, 66, 76).

11. Pansement comprenant un film selon l'une quelconque des revendications précédentes, comportant un tampon d'un matériau absorbant disposé sur la surface interne recouverte d'adhésif du film flexible (1, 21, 28, 40, 51, 57) pour former un pansement à îlot.

12. Pansement comprenant un film selon l'une quelconque des revendications précédentes, dans lequel une partie d'une surface interne de la languette (7, 16, 17, 31, 32, 44, 46, 54, 62) qui fait face à la couche de support (6, 22, 33, 47, 60) comprend une portion de surface non collante à laquelle l'adhésif (4, 20, 27, 39, 50, 58) du film flexible n'adhère pas.

13. Pansement comprenant un film selon la revendication 12, dans lequel la portion de surface non collante se trouve entre la zone d'interface et un bord du pansement comprenant un film.

14. Pansement comprenant un film selon l'une quelconque des revendications 12-13, dans lequel la surface non collante comprend une couche de silicone.

15. Pansement comprenant un film selon l'une quelconque des revendications précédentes, dans lequel la languette (7, 16, 17, 31, 32, 44, 46, 54, 62) comprend un fil dont un utilisateur peut se servir pour tirer la languette et séparer ainsi la couche de support (6, 22, 33, 47, 60) du film flexible.

16. Pansement comprenant un film selon l'une quelconque des revendications précédentes, comportant en outre une couche de support amovible comportant une surface interne et une surface externe, la surface interne étant fixée à la surface externe du film flexible.

17. Procédé d'application d'un pansement comprenant un film sur une surface, **caractérisé en ce que** le procédé comprend les étapes consistant à :
- fournir un pansement comprenant un film selon l'une quelconque des revendications 1-16,
- retirer la doublure anti-adhésive (5, 18, 25, 26, 37, 38, 48, 49, 59) du film flexible (1, 21, 28, 40, 51, 57),
- appliquer le film flexible (1, 21, 28, 40, 51, 57) sur la surface, et retirer la couche de support (6, 22, 33, 47, 60) du film flexible (1, 21, 28, 40, 51, 57) en tirant sur la languette (7, 16, 17, 31, 32, 44, 46, 54, 62).
